# FASCICULE DE BREVET EUROPEEN

(11) **EP 1 059 897 B1**
(45) Date de publication et mention de la délivrance du brevet: **28.09.2005**
(21) Numéro de dépôt: 99906318.3
(22) Date de dépôt: 02.03.1999
(51) Int. Cl.: A61F 2/34, A61F 2/30

(54) **IMPLANT ACETABULAIRE OU COTYLOIDIEN MODULAIRE**
MODULARES HÜFTGELENKPFANNENIMPLANTAT
MODULAR ACETABULAR OR COTYLOID IMPLANT

(30) Priorité: 03.03.1998 FR 9802795
(43) Date de publication de la demande: 20.12.2000
(73) Titulaire: Tornier, 38330 Saint-Ismier (FR)
(72) Inventeur: TORNIER, Alain, F-38330 Saint Ismier (FR)
(74) Mandataire: Myon, Gérard Jean-Pierre
(86) Numéro de dépôt international: PCT/FR1999/000459
(87) Numéro de publication internationale: WO 1999/044545

(56) Documents cités:
- EP-A- 0 142 759
- EP-A- 0 551 794
- WO-A-93/08770
- WO-A-95/15734
- WO-A-97/16137
- DE-C- 4 126 837
- FR-A- 2 578 162
- FR-A- 2 651 996
- FR-A- 2 703 583
- FR-A- 2 748 656
- GB-A- 2 159 416
- US-A- 5 326 368
- US-A- 5 480 448

## Description

La présente invention est relative à un implant acétabulaire ou cotyloïdien modulaire destiné à être placé dans une cavité articulaire endommagée pour coopérer notamment avec la tête d'une prothèse fémorale, l'ensemble constituant une prothèse totale de hanche.

Les caractéristiques du préambule de la revendication 1 sont connues du document GB-A-2 159 416.

On connaît des implants acétabulaires réalisés entièrement en matière plastique telle que du polyéthylène qui sont destinés à coopérer avec une tête fémorale naturelle ou artificielle dans une arthroplastie de la hanche. De tels implants sont mis en place en utilisant un ciment qui solidarise lesdits implants avec la cavité osseuse endommagée.

On connaît également des implants acétabulaires comportant un cotyle métallique et un insert en matière plastique qui est logé dans la cavité interne dudit cotyle. Ce dernier peut être fixé à l'intérieur du cotyle humain d'un patient à l'aide soit de ciment, soit directement par l'intermédiaire de vis qui pénètrent dans l'os iliaque.

Lorsque l'implant est cimenté ou vissé dans le cotyle humain d'un patient, la paroi extérieure du cotyle métallique est soit recouverte d'une épaisseur d'hydroxyapatite, soit pourvue d'un état de surface rugueux pour permettre la repousse osseuse et par conséquent la fixation de l'implant.

L'implant acétabulaire à vis comporte un cotyle métallique qui est percé sur sa périphérie d'un certain nombre de trous permettant au chirurgien plusieurs solutions pour la mise en place des vis de fixation.

Dans les cas de destruction de la cavité cotyloïdienne de l'os il est impossible de remettre un implant de type classique tel que décrit ci-dessus.

Ainsi, l'implant acétabulaire ou cotyloïdien suivant la présente invention, est du genre modulaire et permet, lors de destructions majeures de la cavité cotyloïdienne de l'os, de retrouver les repères anatomiques originels et d'assurer une stabilité immédiate par des appuis et des ancrages solides.

De plus il est prévu d'introduire un capital osseux par apport de greffes, afin de remplir les espaces laissés entre l'implant suivant la présente invention et l'os sain.

L'implant acétabulaire ou cotyloïdien modulaire suivant la présente invention comprend un cotyle métallique recevant dans une cavité interne un insert en matière plastique, ledit cotyle comportant sur sa face externe des rainures disposées à partir de son bord périphérique et en direction du pôle pour permettre la fixation de différents éléments indépendants permettant de retrouver les repères anatomiques originels de l'articulation et d'assurer une stabilité immédiate par des appuis et des ancrages solides.

L'implant acétabulaire ou cotyloïdien conforme à l'invention comprend sur la face externe du cotyle métallique des rainures qui présentent chacune un profil conique dont l'extrémité la plus large est tournée du côté du pôle, tandis que l'autre extrémité, la moins large, est dirigée à proximité du bord périphérique pour éviter l'éjection des éléments indépendants lors de l'enfoncement du cotyle métallique dans la cavité acétabulaire.

L'implant acétabulaire ou cotyloïdien comprend des rainures à profil conique en queue d'aronde.

L'implant acétabulaire ou cotyloïdien comprend des rainures présentant chacune un profil en forme de T dont les bords supérieurs et opposés sont inclinés par rapport à l'axe de ladite rainure de manière que l'extrémité la plus large soit tournée du côté du pôle du cotyle métallique, tandis que les bords inférieurs et opposés du T sont parallèles l'un par rapport à l'autre.

L'implant acétabulaire ou cotyloïdien comprend des rainures présentant chacune un profil en forme de T dont les bords inférieurs et opposés sont inclinés par rapport à l'axe de ladite rainure de manière que l'extrémité la plus large soit tournée du côté du pôle du cotyle métallique, tandis que les bords supérieurs et opposés du T sont parallèles l'un par rapport à l'autre.

L'implant acétabulaire ou cotyloïdien comprend des rainures présentant chacune un profil en forme de T dont les bords supérieurs et inférieurs sont inclinés par rapport à l'axe de ladite rainure de manière que l'extrémité la plus large soit tournée du côté du pôle du cotyle métallique.

L'implant acétabulaire ou cotyloïdien suivant la présente invention comprend sur la face externe du cotyle métallique des rainures qui débouchent au niveau du bord périphérique.

En outre, la présente invention est également relative à un cotyle métallique pour implant acétabulaire ou cotyloïdien comportant sur sa face externe des rainures disposées à partir de son bord périphérique et en direction du pôle pour permettre la fixation de différents éléments indépendants tels que des plaques de fixation ou des cales de comblement de manière à positionner et immobiliser ledit cotyle suivant les repères anatomiques originels.

Le cotyle métallique suivant la présente invention comporte des rainures qui présentent chacune un profil conique dont l'extrémité la plus large est tournée du côté du pôle, tandis que l'autre extrémité, la moins large, est dirigée à proximité du bord périphérique pour éviter l'éjection des éléments indépendants lors de l'enfoncement dudit cotyle métallique dans la cavité acétabulaire.

Le cotyle métallique pour implant acétabulaire ou cotyloïdien comprend sur sa face externe des rainures à profil conique en queue d'aronde.

Le cotyle métallique suivant la présente invention comprend sur sa face externe des rainures présentant chacune un profil en forme de T dont les bords supérieurs et opposés sont inclinés par rapport à l'axe de ladite rainure de manière que l'extrémité la plus large soit tournée du côté du pôle dudit cotyle métallique, tandis que les bords inférieurs et opposés du T sont parallèles l'un par rapport à l'autre.

Le cotyle métallique conforme à l'invention comprend sur sa face externe des rainures présentant chacune un profil en forme de T dont les bords inférieurs et opposés sont inclinés par rapport à l'axe de ladite rainure de manière que l'extrémité la plus large soit tournée du côté du pôle dudit cotyle métallique, tandis que les bords supérieurs et opposés du T sont parallèles l'un par rapport à l'autre.

Le cotyle métallique pour implant acétabulaire ou cotyloïdien comprend sur sa face externe des rainures présentant chacune un profil en forme de T dont les bords supérieurs et inférieurs sont inclinés par rapport à l'axe de ladite rainure de manière que l'extrémité la plus large soit tournée du côté du pôle dudit cotyle métallique.

Le cotyle métallique suivant la présente invention comprend sur sa face externe des rainures qui débouchent du côté de son bord périphérique.

La description qui va suivre en regard des dessins annexés, donnés à titre d'exemple non limitatif, permettra de mieux comprendre l'invention, les caractéristiques qu'elle présente et les avantages qu'elle est susceptible de procurer:
Figure 1 est une vue en perspective illustrant le cotyle métallique et l'insert de l'implant acétabulaire ou cotyloïdien suivant la présente invention.
Figure 2 est une coupe représentant le cotyle métallique de l'implant acétabulaire ou cotyloïdien sans les éléments indépendants.
Figures 3a à 3e sont des vues en perspective montrant les éléments indépendants venant se fixer sur la face externe du cotyle métallique.
Figures 4a et 4b sont des vues illustrant le profil conique en queue d'aronde des rainures ménagées sur la face externe du cotyle métallique pour la fixation des éléments indépendants.
Figures 5a et 5b sont des vues représentant une première variante du profil conique des rainures recevant les éléments indépendants.
Figures 6a et 6b sont des vues montrant une seconde variante du profil conique des rainures recevant les éléments indépendants.
Figures 7a et 7b sont des vues illustrant une troisième variante du profil conique des rainures recevant les éléments indépendants.
Figure 8 est une vue de dessus représentant le cotyle métallique à l'intérieur de la cavité acétabulaire de l'os coxal muni, par exemple, de l'élément indépendant de figures 3a ou 3b.
Figure 9 est une vue de dessus représentant le cotyle métallique à l'intérieur de la cavité acétabulaire de l'os coxal muni, par exemple, de l'élément indépendant de figure 3c.
Figure 10 est une vue de dessus représentant le cotyle métallique à l'intérieur de la cavité acétabulaire de l'os coxal muni, par exemple, de l'élément indépendant de figure 3d.
Figure 11 est une vue de dessus représentant le cotyle métallique à l'intérieur de la cavité acétabulaire de l'os coxal muni, par exemple, de l'élément indépendant de figure 3e.

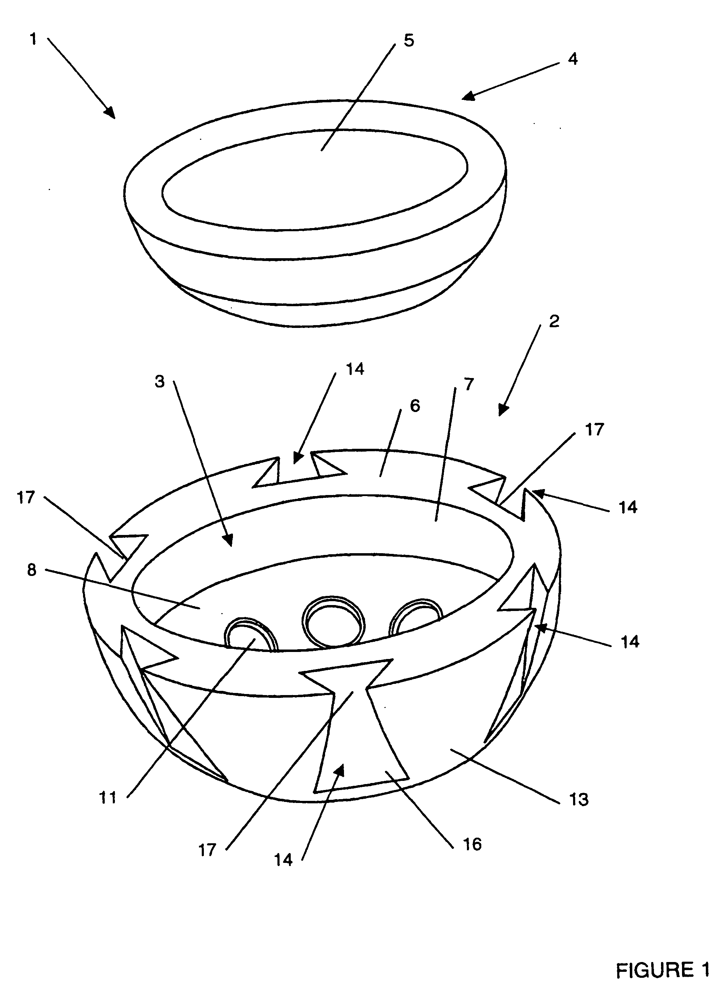

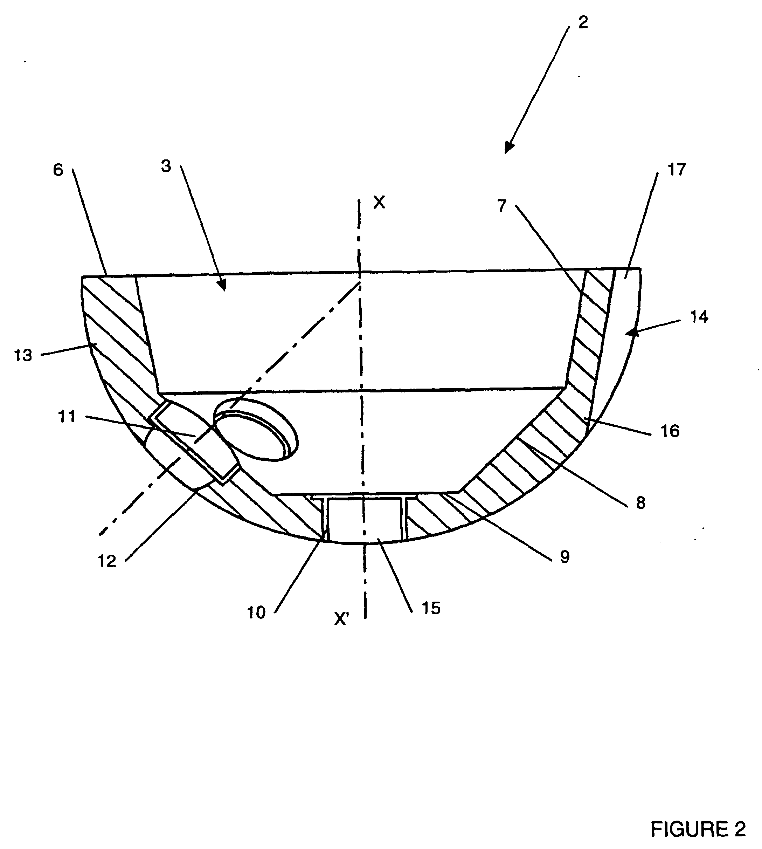

On a montré en figures 1 et 2 un implant acétabulaire ou cotyloïdien 1 comportant un cotyle métallique 2 pourvu d'une cavité interne 3 dans laquelle vient s'engager un insert en matière plastique 4 destiné, par exemple, à recevoir dans son logement 5 une boule d'un implant fémoral non représenté.

Le cotyle métallique 2 présente un profil extérieur hémisphérique destiné à venir se fixer dans la cavité acétabulaire A de l'os coxal C qui peut être préalablement aménagée suivant l'état osseux.

Le cotyle métallique 2 peut être réalisé en acier inoxydable ou en alliage de titane allié ou en tout autre matériau biocompatible.

L'insert 4 destiné à recevoir la boule d'un implant fémoral peut être réalisé en une matière plastique biocompatible telle qu'un polyéthylène haute densité ou céramique.

Le cotyle métallique 2 comporte un bord périphérique 6 délimitant l'entrée de la cavité interne 3. Cette dernière présente, par exemple, perpendiculairement au bord périphérique 6, une première portion de cône à faible conicité 7 qui se prolonge par une autre portion à profil conique 8 de forte conicité.

La portion à profil conique 8 se termine par un fond plat 9 qui est percé en son milieu d'un trou débouchant 10 comportant une partie fileté. Le trou débouchant 10 est porté par l'axe vertical XX' passant par le pôle 15 du cotyle 2.

Il va de soi que le profil interne de la cavité 3 peut être différent sans pour cela changer l'objet de la présente invention.

Le bord périphérique 6 peut être solidaire de manière connue d'ergots, non représentés, qui sont régulièrement répartis sur le pourtour du cotyle métallique 2 pour permettre l'indexation angulaire de l'insert en matière plastique 4 par rapport audit cotyle.

La cavité interne 3 est percée de plusieurs trous débouchant 11 permettant la mise en place de vis de fixation non représentées. Dans notre exemple, les trous 11 sont percés sur un même rayon disposé dans la portion à profil conique 8 de la cavité interne 3. Le cotyle métallique 2 peut comporter un plus grand nombre de trous 11 répartis sur des rayons différents.

Chaque trou 11 comporte un logement 12 à profil sensiblement conique ou en creux permettant de régler la position angulaire de l'axe de la vis de fixation et de recevoir le profil externe de sa tête pour que cette dernière soit la plus éloignée possible de la cavité 3 pour ne pas venir en contact avec l'insert en matière plastique 4.

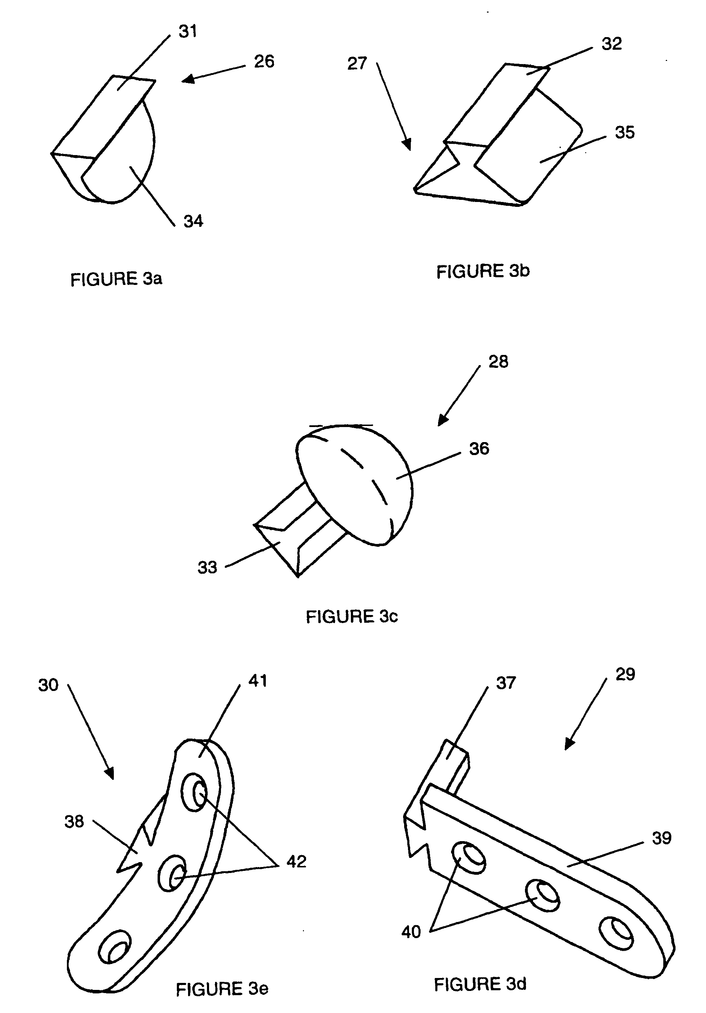

Le cotyle métallique 2 comporte une face externe 13 qui présente des rainures 14 à profils identiques régulièrement réparties sur le pourtour dudit cotyle pour la fixation d'éléments indépendants 26, 27, 28, 29 et 30 montré en figures 3a à 3e.

Chaque rainure 14 est disposée sur axe ou longitude passant par le pôle 15 du cotyle métallique 2. En effet chaque rainure 14 s'étend depuis le bord périphérique 6 en direction du pôle 15 du cotyle 2.

Les rainures 14 présentent chacune un profil conique dont l'extrémité 16, la plus large, est tournée du côté du pôle 15, tandis que l'autre extrémité 17, la moins large, est dirigée à proximité du bord périphérique 6.

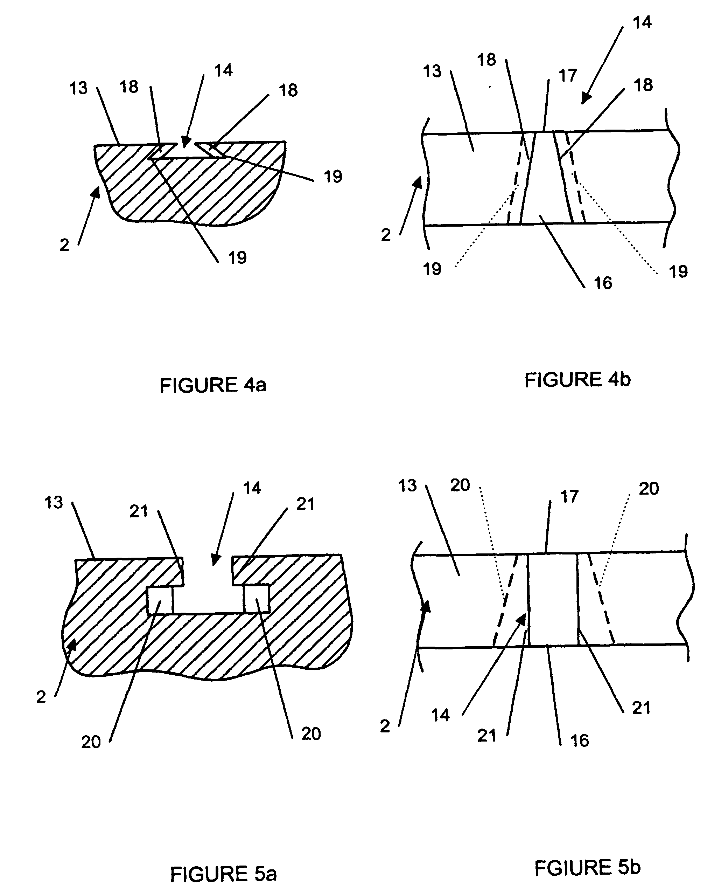

En figures 4a et 4b on a montré une coupe partielle du cotyle métallique 2 qui comporte sur sa face externe 13 des rainures 14 à profil conique en queue d'aronde dont les bords opposés supérieurs 18 et inférieurs 19 sont inclinés de manière que l'extrémité 16 la plus large soit tournée du côté du pôle 15.

En figures 5a et 5b on a représenté une première variante du profil conique des rainures 14 ménagées sur la face externe 13 du cotyle métallique 2.

Chaque rainure 14 présente un profil en forme de T dont les bords inférieurs et opposés 20 sont inclinés par rapport à l'axe de ladite rainure de manière que l'extrémité 16, la plus large, soit tournée du côté du pôle 15 du cotyle métallique 2, tandis que les bords supérieurs et opposés 21 du T sont parallèles l'un par rapport à l'autre.

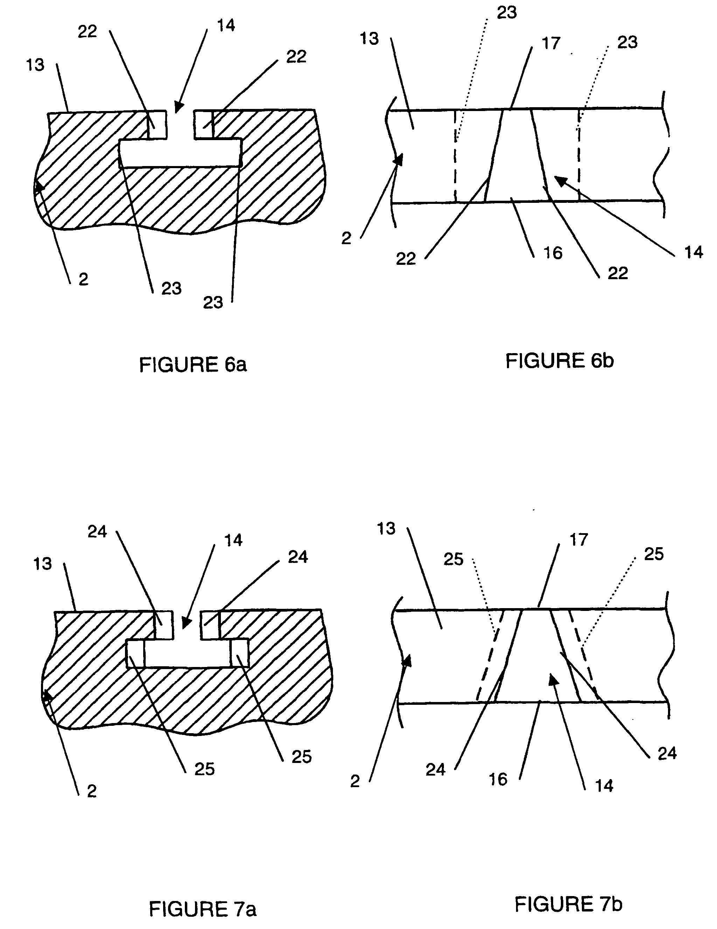

En figures 6a et 6b on a illustré une seconde variante du profil conique des rainures 14 ménagées sur la face externe 13 du cotyle métallique 2.

Chaque rainure 14 présente un profil en forme de T dont les bords supérieurs et opposés 22 sont inclinés par rapport à l'axe de ladite rainure de manière que l'extrémité 16, la plus large, soit tournée du côté du pôle 15 du cotyle métallique 2, tandis que les bords inférieurs et opposés 23 du T sont parallèles l'un par rapport à l'autre.

En figures 7a et 7b on a montré une troisième variante du profil conique des rainures 14 ménagées sur la face externe 13 du cotyle métallique 2.

Chaque rainure 14 présente un profil en forme de T dont les bords supérieurs 24 et inférieurs 25 sont inclinés par rapport à l'axe de ladite rainure de manière que l'extrémité 16, la plus large, soit tournée du côté du pôle 15 du cotyle métallique 2.

On remarque que pour chaque variante de profil de la rainure 14 l'extrémité 16, la plus large, est tournée du côté du pôle 15, positionnant obligatoirement l'autre extrémité 17, la moins large, du côté du bord périphérique 6 du cotyle métallique 2.

Également le cotyle métallique 2 peut comporter sur sa face externe 13 des rainures 14 à profil conique qui débouchent ou non du côté de son bord périphérique 6. Le profil des rainures 14 peut être conique ou non pourvu qu'il puisse permettre le blocage par coincement sur la face externe 13 du cotyle métallique 2 des éléments indépendants 26, 27, 28, 29 et 30.

On a représenté en figures 3a à 3c les éléments indépendants 26, 27 et 28 formant des cales lorsque la cavité acétabulaire A de l'os coxal C est détruite et qu'il est nécessaire de chercher des appuis stables pour la mise en place du cotyle métallique 2.

Les éléments 26 à 28 comportent respectivement un tenon 31, 32 et 33 présentant un profil externe qui est complémentaire à celui des rainures 14. Le profil de chaque tenon dépend de celui que va présenter la rainure 14 correspondante en fonction des solutions décrites ci-dessus afin de pouvoir bloquer par coincement les éléments 26 à 28 sur la face externe 13 du cotyle métallique 2.

Le tenon 31 de l'élément 26 est solidaire d'un disque 34 en portion de cercle qui est parallèle à l'axe longitudinal dudit tenon. Les dimensions du disque 34 peuvent varier selon l'espace E de la cavité acétabulaire A à combler.

Le tenon 32 de l'élément 27 est surmonté d'une pièce 35 à profil triangulaire dont la base la plus large se trouve à l'opposer dudit tenon. Les dimensions de la pièce 35 peuvent varier selon l'espace E de la cavité acétabulaire A à combler.

Le tenon 33 de l'élément 28 est solidaire d'une coquille 36 en portion de sphère qui est disposée à une certaine distance dudit tenon. Les dimensions de la coquille 36 peuvent varier selon l'espace E de la cavité acétabulaire A à combler.

En figures 3d et 3e on a montré deux autres éléments indépendants 29 et 30 permettant la fixation du cotyle métallique 2 contre l'os coxal C lorsque les vis traversant les trous 11 ne sont pas suffisantes pour la stabilité de l'implant 1 dans la cavité acétabulaire A.

Les éléments 29 et 30 comportent respectivement un tenon 37 et 38 coopérant avec la rainure 14 correspondante du cotyle 2. Le profil extérieur de chaque tenon 37, 38 est complémentaire de celui de la rainure 14 du cotyle 2.

Le tenon 37 de l'élément indépendant 29 est solidaire à l'une de ses extrémités d'une plaque 39 rectiligne et percée de trous 40 pour le passage des vis de fixation dans l'os coxal C. La plaque 39 est disposée perpendiculairement au tenon 37 de l'élément 29 (figure 3d).

Le tenon 38 de l'élément indépendant 30 est solidaire à l'une de ses extrémités d'une plaque 41 à profil courbe et percée de trous 42 pour le passage des vis de fixation dans l'os coxal C. La plaque 41 coopère par son milieu avec le tenon 37 afin que le profil courbe soit de distance identique de part et d'autre dudit tenon.

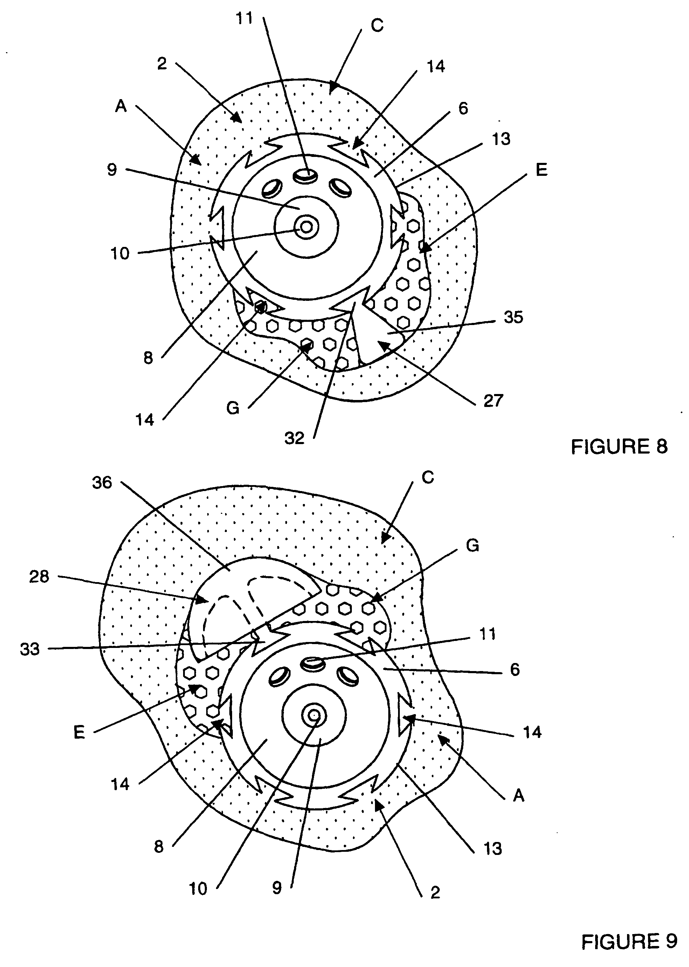

En figures 8 et 9 on a représenté la mise en place du cotyle métallique 2 à l'intérieur de la cavité acétabulaire A de l'os coxal C et dont une partie de l'os à été préalablement retiré par le chirurgien du fait de sa détérioration.

Dans ses conditions le chirurgien vient fixer dans une ou plusieurs rainures 14 de la face externe du cotyle 2, un ou plusieurs des éléments 26, 27 et 28 formant cale, et plus particulièrement celui ou ceux qui permettront de positionner le mieux possible ledit cotyle par rapport aux repères anatomiques originels de l'articulation.

De plus le chirurgien remplira l'espace libre E laissé entre la face externe 13 du cotyle 2 et l'os sain de la partie coxal C d'un greffon osseux G pour améliorer dans le temps la stabilité et l'ancrage de l'implant 1.

Le cotyle 2 sera fixé à l'os soit avec ou sans l'intermédiaire de vis traversant les trous 11 à la manière connue, soit au moyen d'un ou plusieurs autres éléments 29, 30 préalablement ancrés dans les rainures 14 correspondantes.

L'introduction de chaque élément indépendant 26 à 30 dans la rainure 14 correspondante du cotyle 2 s'effectue à partir du pôle 15 et en direction du bord périphérique 6 jusqu'au blocage du tenon 31 à 38 dans ladite rainure.

Le profil conique de chaque rainure 14 dont l'extrémité 16 la plus large est tournée du côté du pôle 15, tandis que l'autre extrémité 17, la moins large, est dirigée à proximité du bord périphérique 6 permet d'éviter l'éjection des éléments indépendants 26 à 30 lors de l'enfoncement dudit cotyle métallique 2 dans la cavité acétabulaire A.

Le cotyle 2 solidaire d'un ou plusieurs éléments indépendants 26, 27 et 28 formant cale constitue avec l'insert en matière plastique 4 un implant 1 dit de reprise. L'implant de reprise 1 est utilisé lors de destructions majeures de la cavité acétabulaire A de l'os coxal C afin de retrouver les repères anatomiques originels et d'assurer une stabilité immédiate par des appuis et des ancrages solides.

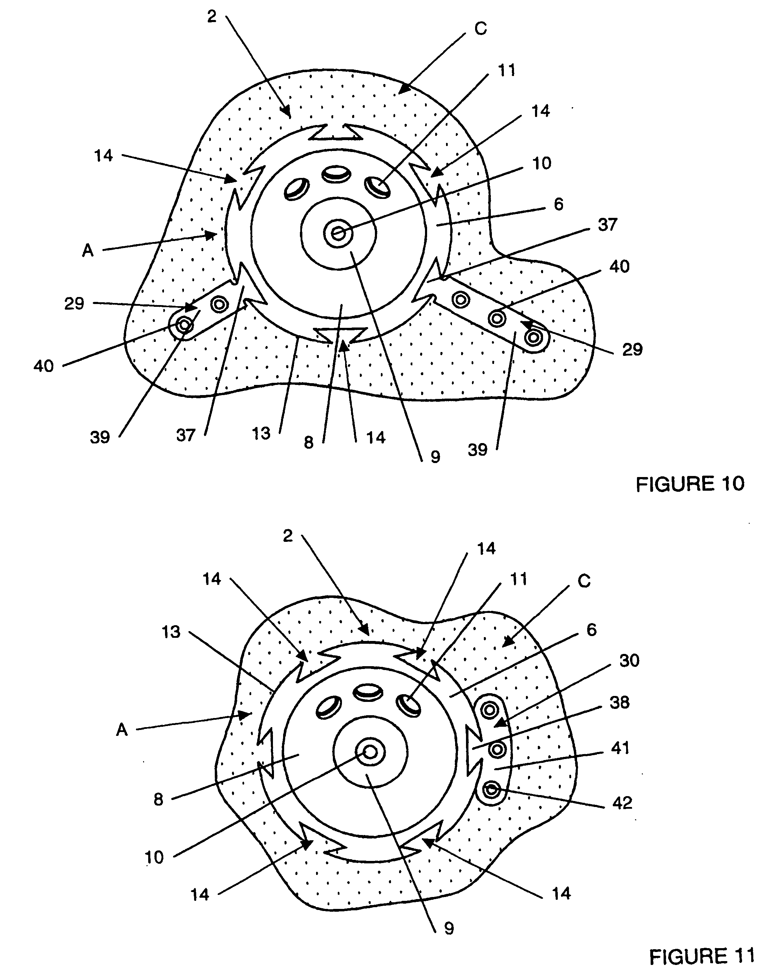

En figures 10 et 11 on a montré la mise en place du cotyle métallique 2 dans la cavité acétabulaire A qui ne comporte pas de destructions importantes obligeant l'emploi des éléments indépendants 26 à 28. Ainsi l'implant 1 est utilisé comme un implant de type classique.

Le cotyle 2 peut être fixé dans la cavité A de l'os coxal C directement au moyen de vis traversant les trous 11 ménagés dans la portion de cône 8 de la cavité interne 3.

Dans le cas ou la stabilisation du cotyle 2 ne serait pas satisfaisante le chirurgien peut compléter l'ancrage au moyen des éléments 29 et 30. Ces derniers sont fixés sur la face externe 13 du cotyle 2 au moyen des rainures 14 comme décrit précédemment.

Enfin, le cotyle métallique 2 peut être fixé dans la cavité A uniquement par les éléments indépendants 29 et 30.

On constate que l'implant 1 suivant la présente invention est du genre modulaire puisqu'il peut recevoir sur la face externe 13 du cotyle métallique 2 différents éléments indépendants 26 à 30 permettant de constituer suivant les cas opératoires, soit un implant de reprise, soit un implant de type classique.

## Revendications

1. Implant acétabulaire ou cotyloïdien comportant un cotyle métallique (2) recevant dans une cavité interne (3) un insert en matière plastique (4), **caractérisé en ce que** le cotyle (2) comporte sur sa face externe (13) des rainures (14) disposées à partir de son bord périphérique (6) et en direction du pôle (15) pour permettre la fixation de différents éléments indépendants (26, 27, 28, 29, 30) permettant de retrouver les repères anatomiques originels de l'articulation et d'assurer une stabilité immédiate par des appuis et des ancrages solides et **en ce que** les rainures (14) présentent chacune un profil conique dont l'extrémité la plus large (16) est tournée du côté du pôle (15), tandis que l'autre extrémité (17), la moins large, est dirigée en direction du bord périphérique (6) pour éviter l'éjection des éléments indépendants (26, 27, 28, 29, 30) lors de l'enfoncement du cotyle métallique dans la cavité acétabulaire.

2. Implant acétabulaire ou cotyloïdien suivant la revendication 1, **caractérisé en ce que** les rainures (14) présentent chacune un profil en forme de T dont les bords supérieurs et opposés (22) sont inclinés par rapport à l'axe de ladite rainure de manière que l'extrémité la plus large (16) soit tournée du côté du pôle (15) du cotyle métallique (2), tandis que les bords inférieurs et opposés (23) du T sont parallèles l'un par rapport à l'autre.

3. Implant acétabulaire ou cotyloïdien suivant la revendication 1, **caractérisé en ce que** les rainures (14) présentent chacune un profil en forme de T dont les bords inférieurs et opposés (20) sont inclinés par rapport à l'axe de ladite rainure de manière que l'extrémité la plus large (16) soit tournée du côté du pôle (15) du cotyle métallique (2), tandis que les bords supérieurs et opposés (21) du T sont parallèles l'un par rapport à l'autre.

4. Implant acétabulaire ou cotyloïdien suivant la revendication 1, **caractérisé en ce que** les rainures (14) présentent chacune un profil en forme de T dont les bords supérieurs (24) et inférieurs (25) sont inclinés par rapport à l'axe de ladite rainure de manière que l'extrémité la plus large (16) soit tournée du côté du pôle (15) du cotyle métallique (2).

5. Implant acétabulaire ou cotyloïdien suivant la revendication 1, **caractérisé en ce que** la face externe (13) du cotyle métallique (2) comporte des rainures (14) qui débouchent du côté du bord périphérique (6).

6. Implant acétabulaire ou cotyloïdien suivant la revendication 1, **caractérisé en ce que** la face externe (13) comporte des rainures (14) qui présentent un profil conique en queue d'aronde.

7. Implant acétabulaire ou cotyloïdien suivant la revendication 1, **caractérisé en ce que** l'implant comprend les éléments indépendants (26, 27, 28, 29, 30) qui comportent respectivement des tenons (31, 32, 33, 37, 38) présentant un profil complémentaire à celui des rainures (14) du cotyle métallique (2).

8. Implant acétabulaire ou cotyloïdien suivant la revendication 7, **caractérisé en ce que** le tenon (31) de l'élément (26) est solidaire d'un disque (34) en portion de cercle.

9. Implant acétabulaire ou cotyloïdien suivant la revendication 8, **caractérisé en ce que** le disque (34) est parallèle à l'axe longitudinal du tenon (31) de l'élément (26).

10. Implant acétabulaire ou cotyloïdien suivant la revendication 7, **caractérisé en ce que** le tenon (32) de l'élément (27) est solidaire d'une pièce à profil triangulaire (35) dont la base la plus large se trouve à l'opposé dudit tenon.

11. Implant acétabulaire ou cotyloïdien suivant la revendication 7, **caractérisé en ce que** le tenon (33) de l'élément (28) est solidaire d'une coquille (36) en portion de sphère.

12. Implant acétabulaire ou cotyloïdien suivant la revendication 7, **caractérisé en ce que** le tenon (37) de l'élément (29) est solidaire d'une plaque (39) rectiligne et percée de trous (40) pour le passage de vis de fixation dans l'os coxal (C).

13. Implant acétabulaire ou cotyloïdien suivant la revendication 12, **caractérisé en ce que** la plaque (39) est disposée perpendiculairement au tenon (37) de l'élément (29).

14. Implant acétabulaire ou cotyloïdien suivant la revendication 7, **caractérisé en ce que** le tenon (38) de l'élément (30) est solidaire à l'une de ses extrémités d'une plaque (41) à profil courbe qui est percée de trous (42) pour le passage de vis de fixation dans l'os coxal (C).

15. Cotyle métallique pour implant acétabulaire ou cotyloïdien suivant la revendication 1, **caractérisé en ce qu'**il comprend sur sa face externe (13) des rainures (14) disposées à partir de son bord périphérique (6) et en direction du pôle (15) pour permettre la fixation de différents éléments indépendants (26, 27, 28, 29, 30) permettant de retrouver les repères anatomiques originels de l'articulation et d'assurer une stabilité immédiate par des appuis et des ancrages solides et **en ce que** les rainures (14) présentent chacune un profil conique dont l'extrémité la plus large (16) est tournée du côté du pôle (15), tandis que l'autre extrémité (17), la moins large, est dirigée en direction du bord périphérique (6) pour éviter l'éjection des éléments indépendants (26, 27, 28, 29, 30) lors de l'enfoncement du cotyle métallique dans la cavité acétabulaire.

16. Cotyle métallique suivant la revendication 15, **caractérisé en ce que** la face externe comporte des rainures qui présentent un profil conique en queue d'aronde.

17. Cotyle métallique suivant la revendication 15, **caractérisé en ce que** les rainures (14) présentent chacune un profil en forme de T dont les bords supérieurs et opposés (22) sont inclinés par rapport à l'axe de ladite rainure de manière que l'extrémité la plus large (16) soit tournée du côté du pôle (15) dudit cotyle métallique, tandis que les bords inférieurs et opposés (23) du T sont parallèles l'un par rapport à l'autre.

18. Cotyle métallique suivant la revendication 15, **caractérisé en ce que** les rainures (14) présentent chacune un profil en forme de T dont les bords inférieurs et opposés (20) sont inclinés par rapport à l'axe de ladite rainure de manière que l'extrémité la plus large (16) soit tournée du côté du pôle (15) dudit cotyle métallique, tandis que les bords supérieurs et opposés (21) du T sont parallèles l'un par rapport à l'autre.

19. Cotyle métallique suivant la revendication 15, **caractérisé en ce que** les rainures (14) présentent chacune un profil en forme de T dont les bords supérieurs (24) et inférieurs (25) sont inclinés par rapport à l'axe de ladite rainure (14) de manière que l'extrémité la plus large (16) soit tournée du côté du pôle (15) dudit cotyle métallique.

20. Cotyle métallique suivant la revendication 15, **caractérisé en ce que** les rainures (14) débouchent du côté du bord périphérique (6).

## Patentansprüche

1. Hüftgelenkpfannen- oder Gelenkpfannenimplantat, das eine metallische Gelenkpfanne (2) trägt, die in einer inneren Höhlung (3) einen Einsatz (4) aus Kunststoffmaterial aufnimmt, **dadurch gekennzeichnet, dass** die Gelenkpfanne (2) auf ihrer Außenseite (13) Einschnitte (14) trägt, die ausgehend von ihrem peripheren Rand (6) und in Richtung zum Pol (15) angeordnet sind, um die Befestigung von verschiedenen unabhängigen Elementen (26, 27, 28, 29, 30) zu ermöglichen, welche eine Rückgewinnung der ursprünglichen anatomischen Fixpunkte des Gelenkes gestatten, und um eine sofortige Stabilität durch Auflagen und feste Verankerungen zu gewährleisten, und dadurch, dass die Einschnitte (14) jeweils ein konisches Profil aufweisen, dessen größtes Ende (16) zur Polseite (15) hin zeigt, während das andere, kleinere Ende (17) in Richtung zum peripheren Rand (6) angeordnet ist, so dass das Ausstoßen der unabhängigen Elemente (26, 27, 28, 29, 30) beim Eintauchen der metallischen Gelenkpfanne in die Höhlung der Hüftgelenkpfanne verhindert wird.

2. Hüftgelenkpfannen- oder Gelenkpfannenimplantat nach Anspruch 1, **dadurch gekennzeichnet, dass** die Einschnitte (14) jeweils ein T-förmiges Profil aufweisen, dessen obere und gegenüberliegende Ränder (22) in Bezug auf die Achse des Einschnittes derart geneigt sind, dass das größte Ende (16) zur Polseite (15) der metallischen Gelenkpfanne (2) gerichtet ist, während die unteren und gegenüberliegenden Ränder (23) des T zueinander parallel sind.

3. Hüftgelenkpfannen- oder Gelenkpfannenimplantat nach Anspruch 1, **dadurch gekennzeichnet, dass** die Einschnitte (14) jeweils ein T-förmiges Profil aufweisen, dessen untere und gegenüberliegende Ränder (20) in Bezug auf die Achse des Einschnittes derart geneigt sind, dass das größte Ende (16) zur Polseite (15) der metallischen Gelenkpfanne (2) gerichtet ist, während die oberen und gegenüberliegenden Ränder (21) des T zueinander parallel sind.

4. Hüftgelenkpfannen- oder Gelenkpfannenimplantat nach Anspruch 1, **dadurch gekennzeichnet, dass** die Einschnitte (14) jeweils ein T-förmiges Profil aufweisen, dessen obere Ränder (24) und untere Ränder (25) in Bezug auf die Achse des Einschnittes derart geneigt sind, dass das größte Ende (16) zur Polseite (15) der metallischen Gelenkpfanne (2) gerichtet ist.

5. Hüftgelenkpfannen- oder Gelenkpfannenimplantat nach Anspruch 1, **dadurch gekennzeichnet, dass** die Außenseite (13) der metallischen Gelenkpfanne (2) Einschnitte (14) trägt, die auf der Seite des peripheren Randes (6) münden.

6. Hüftgelenkpfannen- oder Gelenkpfannenimplantat nach Anspruch 1, **dadurch gekennzeichnet, dass** die Außenseite (13) Einschnitte (14) trägt, die ein schwalbenschwanzförmiges konisches Profil aufweisen.

7. Hüftgelenkpfannen- oder Gelenkpfannenimplantat nach Anspruch 1, **dadurch gekennzeichnet, dass** das Implantat unabhängige Elemente (26, 27, 28, 29, 30) umfasst, die jeweils Zapfen (31, 32, 33, 37, 38) tragen, deren Profil zu demjenigen der Einschnitte (14) der metallischen Gelenkpfanne (2) komplementär ist.

8. Hüftgelenkpfannen- oder Gelenkpfannenimplantat nach Anspruch 7, **dadurch gekennzeichnet, dass** der Zapfen (31) des Elementes (26) mit einer Scheibe (34) in einem Kreisabschnitt einstückig ist.

9. Hüftgelenkpfannen- oder Gelenkpfannenimplantat nach Anspruch 8, **dadurch gekennzeichnet, dass** die Scheibe (34) zur Längsachse des Zapfens (31) des Elementes (26) parallel verläuft.

10. Hüftgelenkpfannen- oder Gelenkpfannenimplantat nach Anspruch 7, **dadurch gekennzeichnet, dass** der Zapfen (32) des Elementes (27) mit einem Teil mit dreieckigem Profil (35), dessen größte Basis sich gegenüber des Zapfens befindet, einstückig ist.

11. Hüftgelenkpfannen- oder Gelenkpfannenimplantat nach Anspruch 7, **dadurch gekennzeichnet, dass** der Zapfen (33) des Elementes (28) mit einer Schale (36) in einem Kugelabschnitt einstückig ist.

12. Hüftgelenkpfannen- oder Gelenkpfannenimplantat nach Anspruch 7, **dadurch gekennzeichnet, dass** der Zapfen (37) des Elementes (29) mit einer Platte (39) einstückig ist, die rechteckig und mit Löchern (40) für das Hindurchleiten von Schrauben zur Befestigung im Hüftbein (C) durchbohrt ist.

13. Hüftgelenkpfannen- oder Gelenkpfannenimplantat nach Anspruch 12, **dadurch gekennzeichnet, dass** die Platte (39) senkrecht zum Zapfen (37) des Elementes (29) angeordnet ist.

14. Hüftgelenkpfannen- oder Gelenkpfannenimplantat nach Anspruch 7, **dadurch gekennzeichnet, dass** der Zapfen (38) des Elementes (30) an einem seiner Enden mit einer Platte (41) mit gekrümmtem Profil, die von Löchern (42) für das Hindurchleiten von Schrauben zur Befestigung im Hüftbein (C) durchbohrt ist, einstückig ist.

15. Metallische Gelenkpfanne für ein Hüftgelenkpfannen- oder Gelenkpfannenimplantat nach Anspruch 1, **dadurch gekennzeichnet, dass** sie auf ihrer Außenseite (13) Einschnitte (14) trägt, die ausgehend von ihrem peripheren Rand (6) und in Richtung zum Pol (15) angeordnet sind, so dass die Befestigung von verschiedenen unabhängigen Elementen (26, 27, 28, 29, 30) möglich ist, die eine Rückgewinnung der ursprünglichen anatomischen Fixpunkte des Gelenks und die Gewährleistung einer sofortigen Stabilität durch Auflagen und feste Verankerungen gestatten, und dadurch, dass die Einschnitte (14) jeweils ein konisches Profil aufweisen, dessen größtes Ende (16) zur Polseite (15) hin zeigt, während das andere, kleinere Ende (17) in Richtung zum peripheren Rand (6) angeordnet ist, so dass das Ausstoßen der unabhängigen Elemente (26, 27, 28, 29, 30) beim Eintauchen der metallischen Gelenkpfanne in die Höhlung der Hüftgelenkpfanne verhindert wird.

16. Metallische Gelenkpfanne nach Anspruch 15, **dadurch gekennzeichnet, dass** die Außenseite Einschnitte trägt, die ein schwalbenschwanzförmiges konisches Profil aufweisen.

17. Metallische Gelenkpfanne nach Anspruch 15, **dadurch gekennzeichnet, dass** die Einschnitte (14) jeweils ein T-förmiges Profil aufweisen, dessen obere und gegenüberliegende Ränder (22) in Bezug auf die Achse des Einschnittes derart geneigt sind, dass das größte Ende (16) zur Polseite (15) der metallischen Gelenkpfanne gerichtet ist, während die unteren und gegenüberliegenden Ränder (23) des T zueinander parallel sind.

18. Metallische Gelenkpfanne nach Anspruch 15, **dadurch gekennzeichnet, dass** die Einschnitte (14) jeweils ein T-förmiges Profil aufweisen, dessen untere und gegenüberliegende Ränder (20) in Bezug auf die Achse des Einschnittes derart geneigt sind, dass das größte Ende (16) zur Polseite (15) der metallischen Gelenkpfanne gerichtet ist, während die oberen und gegenüberliegenden Ränder (21) des T zueinander parallel sind.

19. Metallische Gelenkpfanne nach Anspruch 15, **dadurch gekennzeichnet, dass** die Einschnitte (14) jeweils ein T-förmiges Profil aufweisen, dessen obere Ränder (24) und untere Ränder (25) in Bezug auf die Achse des Einschnittes (14) derart geneigt sind, dass das größte Ende (16) zur Polseite (15) der metallischen Gelenkpfanne gerichtet ist.

20. Metallische Gelenkpfanne nach Anspruch 15, **dadurch gekennzeichnet, dass** die Einschnitte (14) auf der Seite des peripheren Randes (6) münden.

## Claims

1. Acetabular or cotyloid implant comprising a metallic cotyle (2) receiving a plastic insert (4) in an internal cavity (3), **characterized in that** the cotyle (2) comprises, on its outer surface (13), grooves (14) arranged starting from its peripheral edge (6) towards the pole (15) to enable the fixing of various independent elements (26, 27, 28, 29, 30) making it possible to locate the original anatomical markers of the articulation and to ensure instant stability by solid supports and anchors, and **in that** the grooves (14) each have a conical profile, the largest end (16) of which is turned towards the pole (15), whereas the other, smallest end (17) is directed towards the peripheral edge (6) to avoid the ejection of the independent elements (26, 27, 28, 29, 30) during the insertion of the metallic cotyle in the acetabular cavity.

2. Acetabular or cotyloid implant according to Claim 1, **characterized in that** the grooves (14) each have a T-shaped profile whose upper and opposite edges (22) are inclined with respect to the axis of said groove, such that the largest end (16) is turned towards the pole (15) of the metallic cotyle (2), whereas the lower and opposite edges (23) of the T are parallel with respect to one another.

3. Acetabular or cotyloid implant according to Claim 1, **characterized in that** the grooves (14) each have a T-shaped profile whose lower and opposite edges (20) are inclined with respect to the axis of said groove, such that the largest end (16) is turned towards the pole (15) of the metallic cotyle (2), whereas the upper and opposite edges (21) of the T are parallel with respect to one another.

4. Acetabular or cotyloid implant according to Claim 1, **characterized in that** the grooves (14) each have a T-shaped profile whose upper (24) and lower (25) edges are inclined with respect to the axis of said groove, such that the largest end (16) is turned towards the pole (15) of the metallic cotyle (2).

5. Acetabular or cotyloid implant according to Claim 1, **characterized in that** the outer surface (13) of the metallic cotyle (2) comprises grooves (14) that open up towards the peripheral edge (6).

6. Acetabular or cotyloid implant according to Claim 1, **characterized in that** the outer surface (13) comprises grooves (14) which have a dovetail conical profile.

7. Acetabular or cotyloid implant according to Claim 1, **characterized in that** the implant comprises the independent elements (26, 27, 28, 29, 30) which comprise tenons (31, 32, 33, 37, 38), respectively, having a profile that is complementary to that of the grooves (14) of the metallic cotyle (2).

8. Acetabular or cotyloid implant according to Claim 7, **characterized in that** the tenon (31) of the element (26) is affixed to a disc (34) in the form of a half-circle.

9. Acetabular or cotyloid implant according to Claim 8, **characterized in that** the disc (34) is parallel to the longitudinal axis of the tenon (31) of the element (26).

10. Acetabular or cotyloid implant according to Claim 7, **characterized in that** the tenon (32) of the element (27) is affixed to a triangle-shaped piece (35), the largest base of which is opposite said tenon.

11. Acetabular or cotyloid implant according to Claim 7, **characterized in that** the tenon (33) of the element (28) is affixed to a shell (36) in the form of a half-sphere.

12. Acetabular or cotyloid implant according to Claim 7, **characterized in that** the tenon (37) of the element (29) is affixed to a rectilinear plate (39) bored with holes (40) for the passage of fixing screws in the hip bone (C).

13. Acetabular or cotyloid implant according to Claim 12, **characterized in that** the plate (39) is arranged perpendicular to the tenon (37) of the element (29).

14. Acetabular or cotyloid implant according to Claim 7, **characterized in that** the tenon (38) of the element (30) is affixed, at one of its ends, to a curved plate (41) that is bored with holes (42) for the passage of fixing screws in the hip bone (C).

15. Metallic cotyle for an acetabular or cotyloid implant according to Claim 1, **characterized in that** it comprises, on its outer surface (13), grooves (14) arranged starting from its peripheral edge (6) towards the pole (15) to enable the fixing of various independent elements (26, 27, 28, 29, 30) making it possible to locate the original anatomical markers of the articulation and to ensure instant stability by solid supports and anchors, and **in that** the grooves (14) each have a conical profile, the largest end (16) of which is turned towards the pole (15), whereas the other, smallest end (17) is directed towards the peripheral edge (6) to avoid the ejection of the independent elements (26, 27, 28, 29, 30) during the insertion of the metallic cotyle in the acetabular cavity.

16. Metallic cotyle according to Claim 15, **characterized in that** the outer surface comprises grooves that have a dovetail conical profile.

17. Metallic cotyle according to Claim 15, **characterized in that** the grooves (14) each have a T-shaped profile whose upper and opposite edges (22) are inclined with respect to the axis of said groove, such that the largest end (16) is turned towards the pole (15) of said metallic cotyle, whereas the lower and opposite edges (23) of the T are parallel with respect to one another.

18. Metallic cotyle according to Claim 15, **characterized in that** the grooves (14) each have a T-shaped profile whose lower and opposite edges (20) are inclined with respect to the axis of said groove, such that the largest end (16) is turned towards the pole (15) of said metallic cotyle, whereas the upper and opposite edges (21) of the T are parallel with respect to one another.

19. Metallic cotyle according to Claim 15, **characterized in that** the grooves (14) each have a T-shaped profile whose upper (24) and lower (25) edges are inclined with respect to the axis of said groove (14), such that the largest end (16) is turned towards the pole (15) of said metallic cotyle.

20. Metallic cotyle according to Claim 15, **characterized in that** the grooves (14) open up towards the peripheral edge (6).
